# FASCICULE DE BREVET EUROPEEN

(11) **EP 1 890 558 B1**
(45) Date de publication et mention de la délivrance du brevet: **16.02.2011**
(21) Numéro de dépôt: 06755505.2
(22) Date de dépôt: 18.05.2006
(51) Int. Cl.: A23L 1/0532, A23L 1/308, A61K 31/716, A61K 36/03, A61P 1/16

(54) **PRODUITS ALIMENTAIRES CONTENANT DE LA LAMINARINE**
LAMINARIN-ENTHALTENDE LEBENSMITTEL
FOOD PRODUCTS CONTAINING LAMINARIN

(30) Priorité: 18.05.2005 FR 0504984; 19.05.2005 FR 0505040
(43) Date de publication de la demande: 27.02.2008
(73) Titulaire: LABORATOIRES GOEMAR, 35400 Saint-Malo (FR)
(72) Inventeur: YVIN, Jean-Claude, F-35400 Saint-Malo (FR); DELZENNE, Nathalie, B-1332 Genval (BE)
(74) Mandataire: Cabinet Plasseraud
(86) Numéro de dépôt international: PCT/FR2006/001131
(87) Numéro de publication internationale: WO 2006/123060

(56) Documents cités:
- WO-A1-2006/123059
- US-A- 3 081 226
- US-A1- 2003 039 723
- US-A1- 2003 119 780
- US-A1- 2005 065 114
- MICHEL C ET AL: "LES OLIGOSIDES ALGAUX COMME ALIMENTS FONCTIONNELS: ETUDE IN VITRO DE LEURS EFFETS CELLULAIRES ET FERMENTAIRES ALGAL OLIGOSACCHARIDES AS FUNCTIONAL FOODS: IN VITRO STUDY OF THEIR CELLULAR AND FERMENTATIVE EFFECTS" SCIENCES DES ALIMENTS, LAVOISIER ABONNEMENTS, PARIS, FR, vol. 19, no. 3/4, 1999, pages 311-332, XP008055927 ISSN: 0240-8813
- LEE DS: "Production of oligosaccharide from Laminaria japonica by adding the cultured medium of Escherichia coli transformed with vector pLK108s (KFCC-11303) or endo-beta-1,3-glucanase" DERWENT, 5 janvier 2005 (2005-01-05), XP002354749

## Description

L'invention décrit un ingrédient alimentaire.

Elle vise les produits et notamment les aliments le contenant.

Les aliments, outre leur composante minérale, contiennent des glucides, des lipides et des protides. Ces constituants sont métabolisés dans l'organisme et notamment au niveau du foie.

A cet égard on rappelle que le foie est l'organe du corps humain le plus volumineux et doté du métabolisme le plus complexe avec des fonctions excrétrices et de défense interdépendantes.

Le foie a une forte influence sur l'état nutritionnel grâce à son rôle dans le métabolisme intermédiaire des macronutriments, des micronutriments et des sels biliaires.

Un foie malade perturbe la digestion, l'absorption des nutriments et il peut être la cause de carences en vitamines et minéraux ainsi que d'une malnutrition protéolytique.

Certains disfonctionnements du foie participent à l'apparition et au développement d'un fléau moderne, à savoir l'obésité.

Les recommandations des spécialistes de la nutrition visant à inciter les consommateurs à se plier à une hygiène alimentaire propre à protéger le foie et de ce fait contribuer à la lutte contre l'obésité sont loin d'être suivies compte tenu des contraintes qui en résultent et de la tentation exercée par de nombreux aliments recherchés pour leur saveur.

Conférer à ces aliments des propriétés propres à contribuer à la protection, dans le sens évoqué ci-dessus, du tissu hépatique constituerait une avancée importante de la science nutritionnelle.

Le but que la Société Demanderesse s'est fixé a donc été d'apporter une solution à ce problème d'importance toujours croissante.

Or elle a pu trouver que la laminarine, qui est un β-1,3-glucane d'origine marine et qui est couramment utilisée notamment dans l'agriculture où sont mises à profit les propriétés qui lui permettent d'agir sur la croissance des plantes et sur les mécanismes de protection de ces dernières contre certains agents pathogènes, présentait, de façon totalement inattendue et surprenante, la faculté d'exercer un effet protecteur au niveau du tissu hépatique de par son action sur la triglycéridémie et sur les concentrations sériques d'un certain nombre de marqueurs spécifiques d'une souffrance hépatique dont notamment l'alanine aminotransférase.

Des compositions comprenant de la laminarine ont en particulier été décrites dans US 3081226 et US 2003/039723.

L'invention vise des produits ou formulations alimentaires contenant de 2 à 10 % en poids de laminarine par rapport à la masse totale desdits produits alimentaires.

La laminarine peut donc être incorporée dans des formulations alimentaires soit directement soit sous la forme d'un ingrédient alimentaire à base de laminarine, en mélange notamment avec une charge minérale ou organique éventuellement constituée par du carbonate de calcium, du talc, de la silice, du lithothamne, de la maltodextrine ou des dérivés de cellulose.

L'ingrédient alimentaire ainsi constitué comporte de 60 % à 98 % en poids,de préférence de 75 à 95 % en poids de laminarine.

Un ingrédient alimentaire type peut avoir la composition suivante :
Laminarine : 85 % en poids
Charge : 15 % en poids

Sa teneur dans les formulations alimentaires ne dépasse généralement pas 10 % en poids et est, de préférence, de 2 à 8 % en poids par rapport à la masse totale de l'aliment.

Sa teneur dans les produits nutritionnels, constitués de préférence par des comprimés ou gélules, est généralement de 40 à 80 % en poids, le complément 100 étant constitué par des charges minérales et/ou organiques pouvant être choisies dans le groupe comprenant les maltodextrines et les dérivés de cellulose.

Les susdites utilisations de la laminarine sont rendues possibles grâce à son absence de toxicité qui a été démontrée par la Société Demanderesse et grâce au fait qu'elle est sans saveur et ne détériore donc pas le goût des aliments dans lesquels elle est incorporée.

On désigne par le terme laminarine des mélangés de polysaccharides du type β-1,3-glucane, extraits des algues brunes et dont le poids moléculaire est d'environ 2500 à environ 6000.

La laminarine est constituée d'une chaîne principale linéaire de 15 à 35 unités glucopyranose réunies par des liaisons acétaliques β-1,3, cette chaîne principale portant une faible proportion de ramifications.

Le degré de polymérisation moyen est proche de 25.

La laminarine se présente sous une forme soluble et sous une forme insoluble.

Pour l'extraction à partir des algues brunes, on peut se reporter notamment à la demande de brevet internationale WO 03/045414 de la Société Demanderesse, publiée le 5 juin 2003.

La laminarine particulière qui a été utilisée dans le cadre des travaux ayant permis de parvenir à l'invention, avait un poids moléculaire de 4500, un degré de polymérisation moyen de 25, et était constituée essentiellement par des polysaccharides de degrés de polymérisation allant de 20 à 30.

Elle se présentait sous la forme d'une poudre blanc cassé, sans odeur et sans saveur.

Les formulations alimentaires conformes à l'invention peuvent être constituées conformément aux exemples non limitatifs qui suivent.

### Exemple 1

Poudre chocolatée contenant de la laminarine.

| | |
|---|---|
| Cacao dégraissé | 90% |
| Sucre | 2 % |
| Arôme vanille | 0,2% |
| Laminarine | 7,7% |
| Sel de cuisine | 0,1% |

### Exemple 2

| | |
|---|---|
| Poudre de céréales utilisable pour | et contenant |
| Semoule de mais précuite | 80 % |
| Huile d'olive vierge extra | 2,4% |
| Laminarine | 2,1 % |
| Maltodextrine | 2 % |
| Matière grasse végétale hydrogénée (huile de coprah et de palmiste) | 5 % |
| Farine d'épeautre | 1 % |
| Tomates déshydratées | 0,47 % |
| Poudre d'oignon | 0,53 % |
| Huile de tournesol | 1 % |
| Lactose | 5 % |
| Extrait de paprika | 0,5 % |

### Exemple 3

Boisson aromatisée contenant de la laminarine

| | |
|---|---|
| Laminarine | 5% |
| Sucre | 2 % |
| Arôme citron ver | 1 % |
| Eau de source QSP | 100 % |

## Revendications

1. Produit alimentaire contenant de 2 à 10 % en poids de laminarine par rapport à la masse totale du produit alimentaire.

2. Produit alimentaire selon la revendication 1, pour une utilisation pour protéger le tissu hépatique.

3. Produit alimentaire selon la revendication 1 ou la revendication 2, ledit produit étant une poudre chocolatée présentant la composition suivante :
- 90% de cacao dégraissé,
- 2% de sucre,
- 0,2% d'arôme vanille,
- 7,7% de laminarine,
- 0, 1 % de sel de cuisine.

4. Produit alimentaire selon la revendication 1 ou la revendication 2, ledit produit étant une boisson aromatisée présentant la composition suivante :
- 5% de laminarine,
- 2% de sucre,
- 1% d'arôme citron vert,
- de l'eau en quantité suffisante pour 100%.

## Claims

1. A food product comprising 2 to 10% by weight of laminarin based on the total weight of the food product.

2. The food product according to claim 1, for use for protecting the hepatic tissue.

3. The food product according to claim 1 or claim 2, said product being a chocolate powder having the following composition:
- 90% of defatted cocoa,
- 2% of sugar,
- 0.2% of vanilla flavour,
- 7.7% of laminarin,
- 0.1% of salt.

4. The food product according to claim 1 or claim 2, said product being a flavoured beverage having the following composition:
- 5% of laminarin,
- 2% of sugar,
- 1 % of lime flavour,
- water, sufficient quantity for 100%.

## Patentansprüche

1. Lebensmittelprodukt enthaltend 2 bis 10 Gewichtsprozent Laminarin bezogen auf das Gesamtgewicht des Lebensmittelproduktes.

2. Lebensmittelprodukt nach Anspruch 1, zur Verwendung zum Schutz des Lebergewebes.

3. Lebensmittelprodukt nach Anspruch 1 oder Anspruch 2, wobei das besagte Produkt ein Schokolade enthaltendes Pulver ist, welches folgende Zusammensetzung aufweist:
- 90 % entfetteter Kakao,
- 2 % Zucker,
- 0,2 % Vanillearoma,
- 7,7 % Laminarin,
- 0,1 % Kochsalz.

4. Lebensmittelprodukt nach Anspruch 1 oder Anspruch 2, wobei das besagte Produkt ein aromatisiertes Getränk ist, welches folgende Zusammensetzung aufweist:
- 5 % Laminarin,
- 2 % Zucker,
- 1 % Limonenaroma,
- Wasser bis 100 %.
